# EUROPEAN PATENT APPLICATION

(11) **EP 4 442 814 A2**
(43) Date of publication of application: **09.10.2024**
(21) Application number: 22914947.1
(22) Date of filing: 28.12.2022
(51) Int. Cl.: C12N 5/0783, A61K 35/14, A61P 31/12, A61P 35/00, A61P 37/00

(54) **ACTIVATED LYMPHOCYTE EXPANSION METHOD HAVING STABLE AND CONTROLLABLE QUALITY, AND ANTI-TUMOR USE THEREOF**

(30) Priority: 28.12.2021 CN 202111628086
(71) Applicant: Immunotech Applied Science Limited, Beijing 100176 (CN)
(72) Inventor: WANG, Yu, Beijing 100176 (CN); SUN, Lei, Beijing 100176 (CN); SUN, Xuenan, Beijing 100176 (CN); LI, Xuejiao, Beijing 100176 (CN); ZHANG, Yonghua, Beijing 100176 (CN)
(74) Representative: HGF
(86) International application number: PCT/CN2022/142940
(87) International publication number: WO 2023/125696

(57) **Abstract**

The present disclosure relates to a method for preparing an activated expanded lymphocyte stock solution, the preparation method comprising the following steps:(1) co-culturing, in a serum-free medium, a lymphocyte activator with peripheral blood mononuclear cells (PBMCs) obtained via autologous peripheral whole blood centrifugal extraction, and thus completing lymphocyte culture activation, wherein a starting density of culture activation of the peripheral blood mononuclear cells in the serum-free medium is (0.2-1.6)×10⁶ cells/mL, and the serum-free medium is selected from any one of or a combination of KBM 581 and GT-T551 H3; (2) placing the cultured activated lymphocytes prepared in step (1) into a serum-free medium according to a cell density of (0.5-5)×10⁶ cells/mL, and performing subculturing to prepare lymphocyte activation culture, wherein a subculturing temperature is 37.0°C±1.0°C, and an activation culture environment contains 7.5%±1.0%CO₂; (3) adding a serum-free medium having a volume which is 5 to 10 times of the lymphocyte activation culture to the lymphocyte activation culture obtained via the subculturing in step (2), and performing expansion culture, wherein the number of expansion culture generations is 1 to 5; (4) centrifuging, washing, and collecting the activated expanded lymphocytes. The activated expanded lymphocytes prepared in the present disclosure have the advantages of having high cell expansion efficiency, high cell viability, stable cell activity, a long effective period, safety, efficiency, minimal side effects, etc., and effectively solve a key problem of individualized immune cell tumor treatment technology.

## Description

### TECHNICAL FIELD

The present disclosure relates to the field of biomedicine, in particular to an activated lymphocyte expansion method having stable and controllable quality, and anti-tumor use thereof.

### BACKGROUND

Malignant cancer is one of the major diseases that threaten human health and life, and its incidence tends to increase year by year. Data of *Global Cancer Statistics 2015* show that the number of new cases of cancer is about 14.1 million and the number of deaths is as high as 8.2 million globally 2015. There are 4.29 million new cases of cancer, and 2.81 million cases of death in China in 2015. Surgery, radiotherapy and chemotherapy become three conventional means for tumor therapy, but all have the characteristic of insufficient specificity, resulting in different degrees of damage to normal tissues and organs while eliminating tumor cells. In the 1980s, Rosenberg SA et al. observed that after administering recombinant IL-2 treatment to an animal having a non-immune system tumor, lymphocytes of the animal may cause tumor regression and metastasis. Thereafter, use of cellular immunotherapy in the clinic is rapidly expanding and becomes a fourth mode of comprehensive treatment for cancer. Rosenberg SA et al. summarized in 1988 that IL-2 is combined with LAK cells to treat 214 tumor patients, 16 of whom tumor metastasis is completely regressed, and 26 of whom tumor is reduced by more than 50%, and this therapy presents certain therapeutic effects for patients with metastatic renal cell carcinoma, melanoma, colon cancer and non-Hodgkin lymphoma. LAK cells have weak killing power, require a large amount of infusions in clinical applications, and have a limited expansion capacity; IL-2 (a single dose of 1×10⁵ IU/Kg or [1-6]×10⁶ IU/m²) needs to be used in a large dose while cells are infused; however, a significant toxic reaction, including serious ulcer, kidney failure, etc., which is difficult to tolerate for the patient may occur in the treatment process of the large dose of IL-2. The application of the therapy mainly focuses on a few tumors, such as renal cancer and melanoma, and is gradually not adopted.

The Bone Marrow Transplant Research Center of American Stanford Medical School reported in 1991 that peripheral blood mononuclear cells were stimulated with IFN-γ, IL-2, an anti-CD3 monoclonal antibody and IL-1, induced cells have a strong killing effect on lymphoma cells and have little effect on normal hematopoietic stem cells, and cells obtained by the method are named cytokine-induced killer cells (CIK cells). An effector cell playing an anti-tumor effect is mainly an NKT cell of CD3+CD56+ and has the characteristics of anti-tumor activity of T lymphocytes and non-MHC restriction of an NK cell. Studies have shown that an expansion efficiency, tumor cell killing capability, and in vivo activity of the CIK cells are significantly higher than those of LAK cells. Clinical studies have reported the use of CIK cells for treatment of metastatic renal cancer, colon cancer, non-small cell lung cancer and lymphoma.

To date, non-specific cellular immunotherapy has been used in conjunction with tumor therapy means such as surgery, radiotherapy, chemotherapy, targeted therapy, and immunoactivator therapy, a large number of cases in which the non-specific cellular immunotherapy benefits patient therapy have been clinically observed, including prevention of tumor recurrence, prolonging of patient survival, synergy with chemotherapy, significant improvement of patient quality of life, etc., and thus non-specific immune cells have achieved long life in tumor treatment, have received wide attention and have been widely used.

Yamazaki T et al. reported in The Lancet in 2000 randomized control clinical trial results of using an in-vitro culture system to expand and activate autologous lymphocytes for preventing postoperative tumor recurrence of liver cancer. The study enrolled a total of 150 patients with liver cancer who received radical surgical resection, a treatment group (76) received cellular immunotherapy, and a control group (74) did not receive any adjuvant treatment. The patients in the treatment group received a total of 370 cell reinfusions within the first 6 months, and the average number of lymphocytes refused by each patient was 7.1×10¹⁰, in which CD3+ cells accounted for 78%. A total of 62 adverse effects (including fever, headache, nausea, dizziness, itching, and tachycardia) occurred in 45 patients, and all of the adverse effects were self-limiting. None of the patients have symptoms of the lung or kidney, infections with any signs, liver function failure or autoimmune disorders. After a follow-up for an average of 4.4 years (0.2 to 6.7 years), it was found that compared with the control group, the treatment group significantly reduced the risk of recurrence of the liver cancer by 41% (95% of a confidence interval was 12%-60%, p=0.01). The time at which the first recurrence occurs in the treatment group was significantly later than that in the control group. A recurrence-free rate within 3 years was evaluated as 48% (95% of the confidence interval was 37%-59%) in the treatment group and 33% (95% of the confidence interval was 22%-43%) in the control group. A recurrence-free rate within 5 years was evaluated as 38% (95% of the confidence interval was 22%-54%) in the treatment group and 22% (95% of the confidence interval was 11%-34%) in the control group. A recurrence-free survival rate (p=0.01) and a disease-specific survival rate (p=0.04) for the patients in the treatment group was significantly prolonged. The research results showed that this non-specific immunotherapy greatly prolongs a recurrence-free survival time of the patients with liver cancer after surgery. Japan used this technology for clinical tumor treatment since 2000 years, and about 10,000 patients were treated per year by 2010. In 2007, Korea approved the method to enter phase III clinical trials of liver cancer and glioblastoma therapy. In 2012, Korea approved a non-specific immune cell product Immuncell-LC for marketing, which significantly decrease the postoperative recurrence rate of the liver cancer. The number of single reinfusion cells is defined as a range from 1×10⁹ to 2×10¹⁰.

Expanded activated lymphocytes in serum-free cell culture are prepared from mononuclear cells in autologous peripheral blood of the patients. The main component is expanded activated T-lymphocytes (CD3+ cells), and CD8+ killing T-lymphocytes are used as main functional cells. The average number of CD8+T cells in single reinfusion of the patients is 4.70±1.47×10⁹, and it has shown good tumor treatment activity in clinical practice. The prior art discloses a method for expanding and activating lymphocytes in serum-free culture. However, it is required to optimize extraction and separation of peripheral blood mononuclear cells (PBMCs), a type and proportion of lymphocyte activators, a serum-free medium, etc., so as to improve an expansion multiple, a cell viability and a biological activity of the lymphocytes prepared by the method, and meet the requirements for stable and controllable clinical cell quality and safe and effective treatment.

### SUMMARY OF THE INVENTION

An objective of the present disclosure is to provide a method for preparing an activated expanded lymphocyte stock solution, including the following steps:
(1) extracting peripheral blood mononuclear cells (PBMCs) via centrifugation of autologous peripheral whole blood, and co-culturing the peripheral blood mononuclear cells with a lymphocyte activator in a serum-free medium to perform lymphocyte culture activation, producing cultured activated lymphocytes, wherein the peripheral blood mononuclear cells are cultured and activated in the serum-free medium at a starting density of (0.2-1.6)×10⁶ cells/mL and an activation culture temperature of 37.0°C±1.0°C in an activation culture environment that contains 7.5%±1.0% CO₂, wherein the lymphocyte activator is selected from the group consisting of an anti-human CD2 antibody, an anti-human CD3 antibody, an anti-human CD28 antibody, phytohemagglutinin (PHA) and any combination thereof, or is a carrier coated the antibody, and wherein the serum-free medium is selected from the group consisting of KBM 581, GT-T551 H3 and any combination thereof;
(2) placing the cultured activated lymphocytes prepared in step (1) into a serum-free medium selected from the group consisting of KBM 581, GT-T551 H3 and any combination thereof at a cell density of (0.5-5)×10⁶ cells/mL to perform passage culture for 1 to 5 generations at a temperature of 37.0°C±1.0°C in an activation culture environment that contains 7.5%±1.0% CO₂, producing a lymphocyte activation culture;
(3) adding a serum-free medium selected from the group consisting of KBM 581, GT-T551 H3 and any combination thereof at an amount 5 to 10 times the volume of the lymphocyte activation culture to the lymphocyte activation culture obtained in step (2) to perform expansion culture for 1 to 5 generations at a temperature of 37.0°C±1.0°C in an activation culture environment that contains 7.5%±1.0% CO₂; and
(4) centrifuging, washing, and collecting the activated expanded lymphocytes.

In a preferred technical solution of the present disclosure, the peripheral blood mononuclear cells are cultured and activated in the serum-free medium at a starting density of (0.2-1)×10⁶/mL.

In a preferred technical solution of the present disclosure, the lymphocyte activator is an anti-human CD3 antibody, preferably 2.5 µg/mL-5 µg/mL, with a volume of 8-15 mL, more preferably 2.5 µg/mL-3.8 µg/mL, with a volume of 10-13 mL.

In a preferred technical solution of the present disclosure, said extracting peripheral blood mononuclear cells (PBMCs) via centrifugation of autologous peripheral whole blood comprises the following steps: adding a separation medium and a diluent to whole blood subjected to anti-coagulation treatment, stirring, mixing uniformly to obtain a mixture, adding the mixture to a separation solution, centrifuging at a speed of 1000-3000 rpm for a period of 10-40 min, collecting a cell layer between interfaces, adding a washing solution, centrifuging, washing and collecting cells to obtain the peripheral blood mononuclear cells, wherein the separation medium is selected from the group consisting of a hydroxyethyl starch 40 sodium chloride injection (HES), Percoll, Ficoll-Paque PLUS and any combination thereof, a volume ratio of the whole blood : the diluent is 1:(1-2), the diluent is selected from the group consisting of a sodium chloride injection, a Hank's buffer, a Lactated Ringer's solution, a Dulbecco's phosphate buffer and any combination thereof, the separation solution is selected from the group consisting of a hydroxyethyl starch 40 sodium chloride injection (HES), Ficoll, Lymphoprep, Lymphocyte Separation Media, Cell Separation Media and any combination thereof, an osmotic pressure of the separation solution ranges from 300 mOsmol/kg to 360 mOsmol/kg, and the washing solution is selected from the group consisting of a 0.1% human albumin sodium chloride injection, a Dulbecco's phosphate buffer, a sodium chloride injection and any combination thereof.

In a preferred technical solution of the present disclosure, the centrifugation is performed at a speed of 1500-2500 rpm for a period of 15-30 min, preferably at a speed of 2000-2500 rpm for a period of 20-25 min.

In a preferred technical solution of the present disclosure, the washing is centrifugal washing performed for 1 to 5 times at a speed of 500-2000 rpm for a period of 5-20 min, preferably performed for 2 to 3 times at a speed of 1000-1800 rpm for a period of 10-15 min.

In a preferred technical solution of the present disclosure, a density of cells in the passage culture in step (2) is (1-4)×10⁶/mL, and pH after adding a serum-free medium to a passage culture system is in a range from 7.00 to 7.80.

In a preferred technical solution of the present disclosure, the density of cells in the passage culture in step (2) is (2-3)×10⁶/mL, and pH after adding a serum-free medium to a passage culture system is in a range from 7.02 to 7.76.

In a preferred technical solution of the present disclosure, the osmotic pressure of the separation solution is 310-350 mOsmol/kg, preferably 316-347 mOsmol/kg.

In a preferred technical solution of the present disclosure, the number of passage culture generations is 2 to 3.

In a preferred technical solution of the present disclosure, in step (3), when a density of passage culture cells increases to 1 to 10 times a density of culture activation cells, expansion culture is started, a lymphocyte culture obtained via passage culture in step (2) is added to a serum-free medium with a total volume of 6 to 8 times of the lymphocyte culture for expansion culture, and pH of an expansion culture system is in a range from 6.80 to 7.80.

In a preferred technical solution of the present disclosure, in step (3), when the density of passage culture cells increases to 1.2 to 3 times the density of the culture activation cells, expansion culture is started, and pH of the expansion culture system is in a range from 6.88 to 7.70.

In a preferred technical solution of the present disclosure, the number of expansion culture generations is 2 to 3.

In a preferred technical solution of the present disclosure, the centrifugation in step (4) is performed at a speed of 1000-3000rpm for a period of 1-10min, preferably at a speed of 1500-2800rpm for a period of 2-8min), and more preferably at a speed of 2000-2500rpm for a period of 5-6min.

In a preferred technical solution of the present disclosure, 300-600 IU/mL, preferably 400-500 IU/mL of cytokines IL-2 are optionally added to the serum-free medium.

In a preferred technical solution of the present disclosure, pH in the serum-free medium is in a range from 6.9 to 7.9, preferably in a range from 7.2 to 7.4.

In a preferred technical solution of the present disclosure, a culturing device is selected from the group consisting of an incubator, a shaker, and a bioreactor.

In a preferred technical solution of the present disclosure, an expansion multiple of the activated expanded lymphocytes is greater than or equal to 900 times, preferably greater than or equal to 1,000 times, and more preferably, greater than or equal to 1,100 times.

In a preferred technical solution of the present disclosure, the activated expanded lymphocytes have a viability that greater than or equal to 95%, preferably greater than or equal to 98%.

In a preferred technical solution of the present disclosure, the activated expanded lymphocytes comprise CD8+T cells at an amount of greater than or equal to 1×10⁹, preferably from 1×10⁹ to 2×10¹⁰, and more preferably from 4×10⁹ to 9.5×10⁹.

In a preferred technical solution of the present disclosure, the activated expanded lymphocytes have a biological activity KT₅₀ of less than or equal to 8.5, preferably less than or equal to 4, and more preferably less than or equal to 0.7141.

**Another objective of the present disclosure is to provide a pharmaceutical composition comprising an activated expanded lymphocyte stock solution,** the pharmaceutical composition consists of (1-20)×10⁷/mL activated expanded lymphocytes, 0.5-2% human albumin and saline for injection, and has an activated expanded lymphocyte viability of greater than or equal to 95%, wherein CD8+T cells in the activated expanded lymphocytes are (1-20)×10⁷/mL, and the pharmaceutical composition is free of preservatives and antibiotics.

In a preferred technical solution of the present disclosure, the pharmaceutical composition consists of (2-18)×10⁹/mL activated expanded lymphocyte concentration, 1-1.5% human albumin concentration and saline for injection, and has an activated expanded lymphocyte viability of greater than or equal to 98%, wherein CD8+T cells in the activated expanded lymphocytes are in a range from 1×10⁹ to 2×10¹⁰.

In a preferred technical solution of the present disclosure, a cell expansion multiple of a biological sample is greater than or equal to 900 times, preferably greater than or equal to 1000 times, and more preferably, greater than or equal to 1100 times.

In a preferred technical solution of the present disclosure, the activated expanded lymphocytes have a biological activity KT₅₀ of less than or equal to 8.5, preferably less than or equal to 4, and more preferably less than or equal to 0.7141.

In a preferred technical solution of the present disclosure, the total number of the activated expanded lymphocytes in single reinfusion of a patient is less than or equal to 2×10¹⁰, and a cell viability within a validity period is greater than or equal to 85%.

In a preferred technical solution of the present disclosure, an activated expanded lymphocyte viability of the composition stored at 15-25°C within 12 h is greater than or equal to 85%.

Another objective of the present disclosure is to provide a method for preparing a pharmaceutical composition comprising an activated expanded lymphocyte stock solution, comprising resuspending the activated expanded lymphocyte stock solution in saline for injection containing human albumin.

Another objective of the present disclosure is to provide a method for immunotherapy using the activated expanded lymphocytes of the present disclosure, comprising the following steps:
(1) obtaining a biological sample containing lymphocytes from an individual;
(2) activating, passaging and expanding the lymphocytes by using the preparation method of the present disclosure to obtain the activated expanded lymphocytes; and
(3) reinfusing the activated expanded lymphocytes or a pharmaceutical composition thereof to the individual.

Another objective of the present disclosure is to provide a dosage regimen for immunotherapy using the activated expanded lymphocytes of the present disclosure, comprising the following regimens:
(1) in a first course of treatment, allowing a subject receiving 3-6 intravenous infusions, once per week;
(2) in a second course of treatment, allowing the subject receiving 3-6 intravenous infusions, once every two weeks;
(3) in a third course of treatment, allowing the subject receiving 3-6 intravenous infusions, once every three weeks; and
(4) in a fourth course of treatment, allowing the subject receiving 7-10 intravenous infusions, once every four weeks.

In a preferred technical solution of the present disclosure, the dosage regimen includes following regimens:
(1) in the first course of treatment, allowing the subject receiving 4-5 intravenous infusions, once per week;
(2) in the second course of treatment, allowing the subject receiving 4-5 intravenous infusions, once every two weeks;
(3) in the third course of treatment, allowing the subject receiving 4-5 intravenous infusions, once every three weeks; and
(4) in the fourth course of treatment, allowing the subject receiving 8-9 intravenous infusions, once every four weeks.

In a preferred technical solution of the present disclosure, the number of the activated expanded lymphocytes per intravenous infusion is greater than or equal to 2×10⁸, preferably greater than or equal to (2-10)×10⁹.

**Another objective of the present disclosure is to provide use of the activated expanded lymphocytes of the present disclosure in the preparation of drugs for anti-tumor immunotherapy.**

In a preferred technical solution of the present disclosure, tumor is selected from the group consisting of lung cancer, ovarian cancer, colon cancer, rectal cancer, melanoma, kidney cancer, bladder cancer, breast cancer, liver cancer, lymphoma, malignant hematological disease, brain tumor, head and neck cancer, glioma, gastric cancer, nasopharyngeal cancer, laryngeal cancer, cervical cancer, uterine solid tumor, osteosarcoma, bone cancer, pancreatic cancer, skin cancer, prostate cancer, uterine cancer, anal region cancer, testicular cancer, fallopian tube cancer, endometrial cancer, vaginal cancer, vulva cancer, Hodgkin's disease, non-Hodgkin's lymphoma, esophageal cancer, small intestine cancer, endocrine system cancer, thyroid cancer, parathyroid cancer, adrenal gland cancer, soft tissue sarcoma, urinary tract cancer, penile cancer, chronic or acute leukemia, solid tumors of children, lymphocytic lymphoma, bladder cancer, renal or ureter carcinoma, renal pelvis cancer, central nervous system (CNS) tumor, primary CNS lymphoma, tumor angiogenesis, spinal tumor, brain stem glioma, pituitary adenoma, Kaposi's sarcoma, epidermal carcinoma, squamous cell carcinoma, T-cell lymphoma, environmentally induced cancer and any combination thereof.

In a preferred technical solution of the present disclosure, the lung cancer is selected from any one of or complications of small cell lung cancer, and non-small cell lung cancer.

In a preferred technical solution of the present disclosure, the leukemia is selected from any one of or complications of acute lymphoblastic leukemia, acute myelogenous leukemia, B-cell chronic lymphocytic leukemia, and chronic myelogenous leukemia.

In a preferred technical solution of the present disclosure, the acute myelogenous leukemia is selected from the group consisting of acute myeloblastic leukemia, acute promyelocytic leukemia, acute myelomonocytic leukemia, acute basophilic leukemia, acute eosinophilic leukemia, acute megakaryoblastic leukemia, acute red leukemia, acute panmyelosis with myelofibrosis and any combination thereof.

In a preferred technical solution of the present disclosure, the immunotherapy is selected from the group consisting of anti-tumor immunotherapy, immunotherapy after tumor radical resection and any combination thereof.

In a preferred technical solution of the present disclosure, the immunotherapy is immunotherapy after radical resection of primary hepatocellular carcinoma.

In a preferred technical solution of the present disclosure, the drugs are used for adult patients or child patients.

**Another objective of the present disclosure is to provide use of the activated expanded lymphocytes of the present disclosure combined with any one of surgery, radiotherapy and chemotherapy in anti-tumor or immunotherapy thereof.**

**Another objective of the present disclosure is to provide use of the activated expanded lymphocytes in the preparation of drugs for enhancing antiviral capability.**

**Another objective of the present disclosure is to provide use of the activated expanded lymphocytes in the preparation of drugs for enhancing treatment of autoimmune diseases.**

In a preferred technical solution of the present disclosure, the autoimmune diseases are selected from any one of or complications of systemic lupus erythematosus, rheumatoid arthritis, scleroderma, hyperthyroidism, juvenile diabetes, idiopathic thrombocytopenic purpura, autoimmune hemolytic anemia, ulcerative colitis, and dermatosis.

Unless otherwise indicated, the present disclosure uses an expanded activated lymphocyte biological activity (RTCA, taking an effect-target ratio of 40:1 as an example) to characterize a therapeutic activity of the activated expanded lymphocytes. Digested target cells (e.g., HepG2, etc.) are plated in advance with 100 µl of target cells at a density of 2×10⁵ cells/mL, and an effect-target ratio parameter (e.g., an effect-target ratio of 40:1) is set for baseline measurement. After 16-24 h of plating of the target cells for detection, effector cells (namely, the expanded activated lymphocytes) of a specific density (50 µl of a density of 1.6×10⁷cell/L is added per well) according to the set effect-target ratio, and then data such as cell morphology and proliferation differentiation is tacked dynamically and quantitatively in real time, that is, living cells make contact with a microelectrode in a detection plate to generate a change in electrical impedance. When the effector cells are added into wells containing adherent cells, cells with adherent growth on a surface of the microelectrode cause a change in interface impedance of an adherent electrode, and such change is positively correlated with a change in a cell index. Instrument-specific software converts a resistance signal into a specific cell index, and the software monitors a generated cell index curve in real time so as to reveal states such as adhesion, elongation, growth, death of the cells over different time periods. By analyzing changes in the cell index, a killing efficiency of the effector cells against the target cells (such as HepG2 cells) may be calculated, by means of a killing efficiency curve, KT₅₀ (namely, a time taken for the effector cells to kill 50% of the target cells) is calculated, and a KT₅₀ value is taken as an index for determining the biological activity of the effector cells (namely, the activated expanded lymphocytes). The smaller the KT₅₀ value, the higher the killing efficiency and the higher the biological activity. Otherwise, the biological activity is lower.

Unless otherwise specified, when the present disclosure relates to a percentage between liquid and liquid, the percentage is a volume/volume percentage; when the present disclosure relates to a percentage between liquid and solid, the percentage is a volume/weight percentage; when the present disclosure relates to a percentage between solid and liquid, the percentage is a weight/volume percentage; and the rest is a weight/weight percentage.

Compared with the prior art, the present disclosure has the following beneficial technical effects:
1. The activated expanded lymphocytes prepared in the present disclosure have the advantages of high cell expansion efficiency, high cell viability, stable cell activity, long effective period, safety and effectiveness, and small side effects, the present disclosure effectively solves the key problems of an individualized immune cell tumor treatment technology, first, a serum-free culture system with a stable cell culture efficiency is used, a killing activity and the expansion capability of the cells are always maintained for 12 days of continuous culturing, and pollution of pathogenic microorganisms and other adverse factors on cell growth that may be caused by using the serum are avoided; second, the cell expansion efficiency is high, and it is not necessary to use a mononuclear cell harvester for efficient expansion of more than 1000 times, thereby avoiding a damaging effect of harvesting a large number of initial peripheral blood mononuclear cells by using a single-harvester on the whole immune cell system of a patient in traditional cellular immunotherapy; third, the cell activity is high, stability is good, and the cells may be stored for 12 h without affecting the treatment effect thereof; and fourth, the technical problems of a standardized quality control system and mass production of an individualized product are solved, a technical process of a cell product with a uniform relative quality is obtained, and the treatment targeting and treatment accuracy and patient compliance are significantly improved.
2. After the activated expanded lymphocytes of the present disclosure are used for treating tumor patients, the tumor postoperative recurrence may be prevented, a postoperative recurrence period of the tumor is prolonged, survival of the tumor patients is significantly prolonged and the survival quality of the patients are significantly improved; and in the treatment process, a chemotherapy drug dosage and a radiotherapy dosage are reduced, adverse reactions such as nausea, vomiting, alopecia, and insomnia occurring in a radiotherapy and chemotherapy treatment process are reduced, and there are no serious adverse reactions such as hemogram reduction and liver and kidney function impairment, thereby reducing pain caused by the tumor, and significantly improving the quality of life of the patients.
3. The lymphocyte expansion method of the present disclosure has the advantages of being easy to operate, stable and controllable in quality, suitable for industrial production, etc.

### BRIEF DESCRIPTION OF THE DRAWINGS

FIG. 1 is a growth condition of EAL cells at first passage culture of Embodiment 1 (observation under a 250×microscope);
FIG. 2 is a growth condition of EAL cells at first expansion culture of Embodiment 1 (observation under a 250×microscope);
FIG. 3 is a growth condition of EAL cells at second expansion culture of Embodiment 1 (observation under a 250×microscope).

### DETAILED DESCRIPTION

The present disclosure is further explained below in combination with embodiments, but the present disclosure is not limited to the scope of the described embodiments.

### Embodiment 1 Preparation of Lymphocyte Activated Expanded Stock Solution

### (1) Separation of peripheral blood mononuclear cells (PBMCs) and inoculation and activated culture (day 0)

Patient 1: patient with liver cancer, age: 55 years old, and gender: male.

A serum-free cell medium is a GT-T551 H3 serum-free cell medium (a concentration of IL-2 in the medium is 500 IU/mL, pH is in a range from 7.2 to 7.4)

37 mL of drawn patient whole blood was placed into a new 250 mL centrifuge tube, and a 0.9% sodium chloride injection with a volume equal to that of a blood sample was added; after being mixed uniformly, a mixture was slowly added into a 50 mL centrifuge tube (two centrifuge tubes) which has been assembled with 15 ml of Ficoll, so as to ensure clear separation interfaces; and centrifugation was performed at 2000 rpm for 20 min, and a centrifuge was set to accelerate by 7 and decelerate by 0. A cell layer between the interfaces was sucked into a new 50 mL centrifuge tube, mixed uniformly with a 0.9% sodium chloride injection in a ratio of 1:1, and centrifuged at 1200 rpm for 10 min. After the centrifugation was completed, a supernatant was decanted, and after a cell pellet was shaken off, a mixture was resuspended with 50 mL of 0.9% sodium chloride injection and centrifuged at 1200 rpm for 10 min. After the centrifugation was completed, a supernatant was decanted, after a cell pellet was shaken off, 5 mL of serum-free cell medium was added, and after mixing uniformly, 10 µl of cell suspension was taken for cell counting. After suspending the cells at a concentration of 0.8×10⁶ cells/mL using the serum-free cell medium, the cells were inoculated into a 225cm² cell culture flask coated with an anti-human CD3 antibody, sampled, aseptically examined and labeled, and the cell culture flask was placed in a cell incubator for culturing.

In a preparation process of the lymphocyte activated expanded stock solution, a culture condition of the incubator was 37°C and 7.5% CO₂.

### (2) First passage culture (day 3)

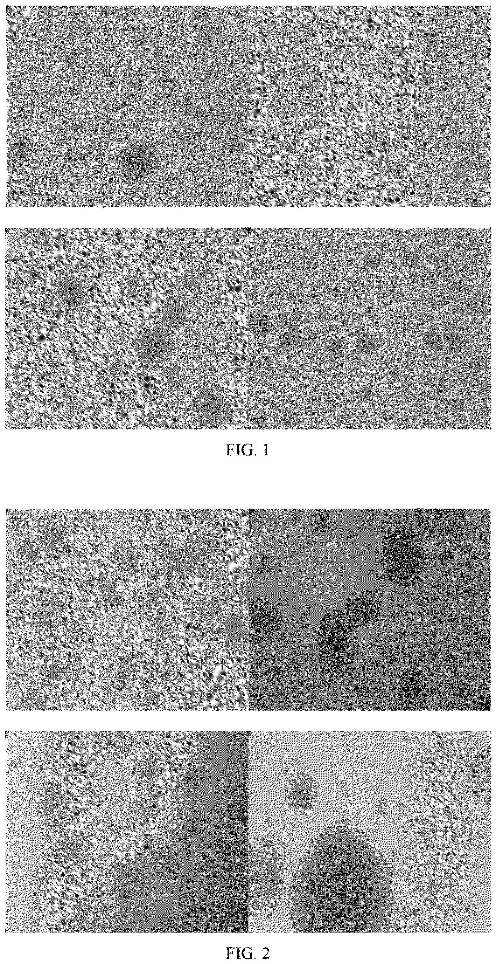

When a color of the medium becomes lighter and yellow (taking the day of blood intake as the day 0, and day 3 after inoculation and culture), the cell culture flask was taken out from the incubator; and the morphology of the cells was observed under a microscope: a growth condition was good (see FIG. 1). 50 mL of serum-free cell medium was added and poured into the cell culture flask, and the cell culture flask was placed back to the cell incubator for culturing.

### (3) Second passage culture (day 4)

When a color of the medium becomes lighter and yellow, the cell culture flask was taken out from the incubator; and the morphology of the cells was observed under a microscope: a growth condition was good. 140 mL of serum-free cell medium was added, and the cell culture flask was placed back to the cell incubator for culturing.

### (4) First expansion culture (day 5)

The cell culture flask was taken out from the incubator, and the morphology of the cells was observed under a microscope: a growth condition was good (see FIG. 2); and samples were taken for cell counting, when a living cell density was not less than 10×10⁶/mL, cell culture liquid and 760 mL of serum-free cell medium were poured into a cell culture bag, and the cell culture flask was placed back into the cell incubator for culturing.

### (5) Second expansion culture (day 8)

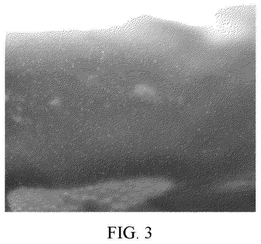

When a color of the culture liquid turned yellow, a new cell culture bag was taken to a bio-safety cabinet, 1000 mL of serum-free cell medium (bag A) was poured through a syringe sleeve, and a tube orifice was sealed. A cell culture bag (bag B) containing cells in culturing was taken out from the incubator, the two bags was connected using a sterile connection device. First, all liquid in the bag A was made to flow into the bag B, uniform mixing was performed, and then 1000 mL of liquid was placed back into the bag A. The liquid volume in the two bags was 1000 mL. The tube orifice was sealed, and a sample was drawn for sterility inspection. The morphology of the cells was observed under a microscope (see FIG. 3), and two bags of cells were placed back to the incubator for culturing.

### (6) Purifying, concentrating by centrifugation and collecting cells to prepare the cell stock solution (day 12)

On day 4 after the two cell culture bags, one cell culture bag was taken out from the incubator to the bio-safety cabinet, the cell suspension was equally divided into four 250 mL centrifuge tubes, and was centrifuged at 2000 rpm for 5 min. A supernatant was decanted, and precipitation was shaken off. Then, the other cell culture bag of the same batch was taken out from the incubator to the bio-safety cabinet, and the cell suspension was equally divided into four original 250 mL centrifuge tubes, and centrifuged at 2000 rpm for 5 min. A supernatant was decanted, and precipitation was shaken off. The cells in one tube was washed for three times with 250 mL of 0.9% sodium chloride injection containing 0.1% human albumin and combined into the other tube, the four tubes were combined into two tubes, and centrifugation was performed at 2000 rpm for 5 min. A supernatant was decanted, and precipitation was shaken off. Then the cells in the two tubes were washed for three times with 200 mL of 0.1% human serum albumin-sodium chloride injection and combined into one tube; after mixing uniformly, a mixture was sampled for cell counting, and centrifuged at 2000 rpm for 5 min; and after a supernatant was decanted, the activated expanded lymphocyte stock solution was obtained, and 2.30×10¹⁰ activated expanded lymphocytes were obtained.

### (7) Preparation of a composition

The activated expanded lymphocytes were obtained by resuspending the cell stock solution at a concentration of 8×10⁷/mL in saline for injection containing 1% human albumin.

### Embodiment 2 Preparation of Lymphocyte Activated Expanded Stock Solution

### (1) Separation of PBMCs and inoculation and activated culture (day 0)

Patient 2: patient with breast cancer, age: 50 years old, and gender: female.

A serum-free cell medium is a KBM 581 serum-free cell medium (a concentration of IL-2 in the medium is 500 IU/mL, pH is in a range from 7.2 to 7.4)

63 mL of whole blood was placed into a new 250 mL centrifuge tube, and a sodium chloride injection with a volume equal to that of a blood sample was added; after being mixed uniformly, a mixture was slowly added into a 50 mL centrifuge tube (four centrifuge tubes) which has been assembled with 15 ml of Ficoll, so as to ensure clear separation interfaces; and centrifugation was performed at 2000 rpm for 20 min, and a centrifuge was set to accelerate by 7 and decelerate by 0. A cell layer between the interfaces was sucked into a new 50 mL centrifuge tube, mixed uniformly with a 0.9% sodium chloride injection in a ratio of 1:1, and centrifuged at 1200 rpm for 10 min. After centrifugation was completed, a supernatant was decanted, and after a cell pellet was shaken off, a mixture was resuspended with 50 mL of sodium chloride injection and centrifuged at 1200 rpm for 10 min. After the centrifugation was completed, a supernatant was decanted, after a cell pellet was shaken off, 5 mL of serum-free cell medium was added, and after mixing uniformly, 10 µl of cell suspension was taken for cell counting. After suspending the cells at a concentration of 0.2×10⁶ cells/mL using the serum-free cell medium, the cells were inoculated into a 225cm² cell culture flask coated with an anti-human CD3 antibody. The cells were sampled, aseptically examined and labeled, and the cell culture flask was placed in a cell incubator for culturing.

In a preparation process of the lymphocyte activated expanded stock solution, a culture condition of the incubator was 37°C and 7.5% CO₂.

### (2) First passage culture (day 4)

When a color of the medium becomes lighter and yellow (taking the day of blood intake as the day 0, and day 4 after inoculation and culture), the cell culture flask was taken out from the incubator; and the morphology of the cells was observed under a microscope: a growth condition was good. Cell counting was performed, 50 mL of serum-free cell medium was added and poured into the cell culture flask, and the cell culture flask was placed back to the cell incubator for culturing.

### (3) Second passage culture (day 5)

When a color of the medium becomes lighter and yellow, the cell culture flask was taken out from the incubator; and the morphology of the cells was observed under a microscope: a growth condition was good. Cell counting was performed, 140 mL of serum-free cell medium was added and poured into the cell culture flask, and the cell culture flask was placed back to the cell incubator for culturing.

### (4) First expansion culture (day 6)

The cell culture flask was taken out from the incubator, and the morphology of the cells was observed under a microscope: a growth condition was good; and samples were taken for cell counting, when a living cell density was not less than 1×10⁶/mL, cell culture liquid and 750 mL of serum-free cell medium were poured into a cell culture bag, and the cell culture flask was placed back into the cell incubator for culturing.

### (5) Second expansion culture (day 8)

When a color of the culture liquid turned yellow, a new cell culture bag was taken to a bio-safety cabinet, 1000 mL of serum-free cell medium (bag A) was poured through a syringe sleeve, and a tube orifice was sealed. A cell culture bag (bag B) containing cells in culturing was taken out from the incubator, and the two bags was connected using a sterile connection device. First, all liquid in the bag A was made to flow into the bag B, uniform mixing was performed, and then 1000 mL of liquid was placed back into the bag A. The liquid volume in the two bags was 1000 mL. The tube orifice was sealed, and a sample was drawn for sterility inspection. The morphology of the cells was observed under a microscope, and two bags of cells were placed back to the incubator for culturing.

### (6) Purifying, concentrating by centrifugation and collecting cells to prepare the cell stock solution (day 12)

On day 4 after the two cell culture bags, one cell culture bag was taken out from the incubator to the bio-safety cabinet, the cell suspension was equally divided into four 250 mL centrifuge tubes, and was centrifuged at 2000 rpm for 5 min. A supernatant was decanted, and precipitation was shaken off. Then, the other cell culture bag of the same batch was taken out from the incubator to the bio-safety cabinet, and the cell suspension was equally divided into four original 250 mL centrifuge tubes, and centrifuged at 2000 rpm for 5 min. A supernatant was decanted, and precipitation was shaken off. The cells in one tube was washed for three times with 250 mL of 0.9% sodium chloride injection and combined into the other tube, the four tubes were combined into two tubes, and centrifugation was performed at 2000 rpm for 5 min. A supernatant was decanted, and precipitation was shaken off. Then the cells in the two tubes were washed for three times with 200 mL of sodium chloride injection and combined into one tube; after mixing uniformly, a mixture was sampled for cell counting, and then centrifuged at 2000 rpm for 5 min; and after a supernatant was decanted, the activated expanded lymphocyte stock solution was obtained, and 2.84×10¹⁰ activated expanded lymphocytes were obtained.

### (7) Preparation of a composition

The activated expanded lymphocytes were obtained by resuspending the cell stock solution at a concentration of 6×10⁷/mL in saline for injection containing 1.5% human albumin.

### Embodiment 3 Preparation of Lymphocyte Activated Expanded Stock Solution

### (1) Separation of PBMCs and inoculation and activated culture (day 0)

Patient 3: patient with non-small cell lung cancer, age: 53 years old, and gender: male.

A serum-free cell medium is a KBM 581 (CORNING) serum-free cell medium (a concentration of IL-2 in the medium is 400 IU/mL, pH is in a range from 7.2 to 7.4)

58 mL of drawn patient whole blood was placed into a new 250 mL centrifuge tube, and a 0.9% sodium chloride injection with a volume equal to that of a blood sample was added; after being mixed uniformly, a mixture was slowly added into a 50 mL centrifuge tube (four centrifuge tubes) which has been assembled with 15 ml of Ficoll, so as to ensure clear separation interfaces; and centrifugation was performed at 2000 rpm for 20 min, and a centrifuge was set to accelerate by 7 and decelerate by 0. A cell layer between the interfaces was sucked into a new 50 mL centrifuge tube, mixed uniformly with a 0.9% sodium chloride injection in a ratio of 1:1, and centrifuged at 1200 rpm for 10 min. After centrifugation was completed, a supernatant was decanted, and after a cell pellet was shaken off, a mixture was resuspended with 50 mL of sodium chloride injection and centrifuged at 1200 rpm for 10 min. After the centrifugation was completed, a supernatant was decanted, after a cell pellet was shaken off, 5 mL of serum-free cell medium was added, and after mixing uniformly, 10 µl of cell suspension was taken for cell counting. After suspending the cells at a concentration of 0.3×10⁶ cells/mL using the serum-free cell medium, the cells were inoculated into a 225cm² cell culture flask coated with an anti-human CD3 antibody. The cells were sampled, aseptically examined and labeled, and the cell culture flask was placed in a cell incubator for culturing.

In a preparation process of the lymphocyte activated expanded stock solution, a culture condition of the incubator was 37°C and 5% CO₂.

### (2) First passage culture (day 3)

When a color of the medium becomes lighter and yellow (taking the day of blood intake as the day 0, and day 3 after inoculation and culture), the cell culture flask was taken out from the incubator; and the morphology of the cells was observed under a microscope: a growth condition was good. Cell counting was performed, 50 mL of serum-free cell medium was added and poured into the cell culture flask, and the cell culture flask was placed back to the cell incubator for culturing.

### (3) Second passage culture (day 4)

When a color of the medium becomes lighter and yellow, the cell culture flask was taken out from the incubator; and the morphology of the cells was observed under a microscope: a growth condition was good. Cell counting was performed, 140 mL of serum-free cell medium was added and poured into the cell culture flask, and the cell culture flask was placed back to the cell incubator for culturing.

### (4) First expansion culture (day 6)

The cell culture flask was taken out from the incubator, and the morphology of the cells was observed under a microscope: a growth condition was good; and samples were taken for cell counting, when a living cell density was not less than 1×10⁶/mL, cell culture liquid and 750 mL of serum-free cell medium were poured into a cell culture bag, and the cell culture flask was placed back into the cell incubator for culturing.

### (5) Second expansion culture (day 8)

When a color of the culture liquid turned yellow, a new cell culture bag was taken to a bio-safety cabinet, 1000 mL of serum-free cell medium (bag A) was poured through a syringe sleeve, and a tube orifice was sealed. A cell culture bag (bag B) containing cells in culturing was taken out from the incubator, and the two bags was connected using a sterile connection device. First, all liquid in the bag A was made to flow into the bag B, uniform mixing was performed, and then 1000 mL of liquid was placed back into the bag A. The liquid volume in the two bags was 1000 mL. The tube orifice was sealed, and a sample was drawn for sterility inspection. The morphology of the cells was observed under a microscope, and two bags of cells were placed back to the incubator for culturing.

### (6) Purifying, concentrating by centrifugation and collecting cells to prepare the cell stock solution (day 12)

On day 4 after the two cell culture bags, one cell culture bag was taken out from the incubator to the bio-safety cabinet, the cell suspension was equally divided into four 250 mL centrifuge tubes, and was centrifuged at 2000 rpm for 5 min. A supernatant was decanted, and precipitation was shaken off. Then, the other cell culture bag of the same batch was taken out from the incubator to the bio-safety cabinet, and the cell suspension was equally divided into four original 250 mL centrifuge tubes, and centrifuged at 2000 rpm for 5 min. A supernatant was decanted, and precipitation was shaken off. The cells in one tube was washed for three times with 250 mL of 0.9% sodium chloride injection and combined into the other tube, the four tubes were combined into two tubes, and centrifugation was performed at 2000 rpm for 5 min. A supernatant was decanted, and precipitation was shaken off. Then the cells in the two tubes were washed for three times with 200 mL of sodium chloride injection and combined into one tube; after mixing uniformly, a mixture was sampled for cell counting, and then centrifuged at 2000 rpm for 5 min; and after a supernatant was decanted, the expanded activated lymphocyte stock solution was obtained, and 2.23×10¹⁰ activated expanded lymphocytes were obtained.

### (7) Preparation of a composition

The activated expanded lymphocytes were obtained by resuspending the cell stock solution at a concentration of 8×10⁷/mL in saline for injection containing 1% human albumin.

### Embodiment 4 Screening of Serum-free Medium

An effect of three serum-free media such as KBM 581 (CORNING), GT-T551 H3 (TAKARA), and TexMACS GMP Medium (Miltenyi biotec) on the preparation of the EAL cells was investigated for in vitro comparison using the same activation expansion process as in embodiment 1.

Peripheral blood of three normal human was collected to separate PBMCs for cell counting and phenotype detection, each PBMC was trisected and then respectively inoculated into a cell culture flask coated with an activated antibody by using three serum-free media for culturing, cell counting was performed once every 4-6 days, the corresponding cell culture medium was supplemented, after 12 days, the culturing was ended, and cell counting, viability detection, phenotype detection and killing activity detection were performed.

A serum-free medium TexMACS GMP Medium was used to culture cells, the cell expansion was slow after 6-8 days of culturing, which cannot meet the quality requirements for lymphocyte expansion in vitro.

**Table 1**

| **Medium** | **Group** | **Label** | **Number of PBMC (×10⁷)** | **Number of cells after 12 days of culture (x10⁷)** |
|---|---|---|---|---|
| KBM 581 | Group A | A1 | **1.0** | **972.00** |
| | | A2 | **1.0** | **952.00** |
| | | A3 | **1.0** | **1100.00** |
| GT-T551 H3 | Group B | B1 | **1.0** | **1448.00** |
| | | B2 | **1.0** | **1288.00** |
| | | B3 | **1.0** | **1116.00** |
| TexMACS GMP Medium | Group C | D1 | **1.0** | **ND** |
| | | D2 | **1.0** | **ND** |
| | | D3 | **1.0** | **ND** |

**Table 2**

| **Group** | **Label** | **PBMC viability (%)** | **Cell viability after 12 days of culture (%)** |
|---|---|---|---|
| Group A | A1 | 100.00 | 99.90 |
| | A2 | 100.00 | 99.90 |
| | A3 | 100.00 | 100.0 |
| Group B | B1 | 100.00 | 99.90 |
| | B2 | 100.00 | 100.0 |
| | B3 | 100.00 | 99.90 |
| Group C | D1 | 100.00 | ND |
| | D2 | 100.00 | ND |
| | D3 | 100.00 | ND |

**Table 3**

| **Group** | **Before culture (%)** | | **After culture (%)** | |
|---|---|---|---|---|
| | **CD3+(%)** | **CD3+CD8+** | **CD3+** | **CD3+CD8+** |
| A1 | 23.12% | 30.35% | 99.61% | 95.68% |
| A2 | 28.03% | 19.60% | 99.29% | 84.24% |
| A3 | 30.30% | 18.22% | 99.31% | 81.16% |
| B1 | 23.12% | 30.35% | 98.96% | 93.89% |
| B2 | 28.03% | 19.60% | 99.04% | 85.05% |
| B3 | 30.30% | 18.22% | 99.41% | 85.81% |
| C1 | 23.12% | 30.35% | ND | ND |
| C2 | 28.03% | 19.60% | ND | ND |
| C3 | 30.30% | 18.22% | ND | ND |

### Embodiment 5: Expansion Effect of Activated lymphocytes in Patients

(1) Peripheral blood from a total of 50 patients were collected, and distributed across different genders, ages, and tumor types. The basic information of the patients was shown in Table 4 to Table 6.

**Table 4**

| Gender | Male | Female | Total |
|---|---|---|---|
| Number | 25 | 25 | 50 |

**Table 5**

| | | | | |
|---|---|---|---|---|
| Age | 21-40 | 41-60 | 61-80 | Total |
| Number of | 5 | 24 | 21 | 50 |
| people | | | | |

**Table 6**

| Tumor type | Number of people |
|---|---|
| Hematologic tumor | 9 |
| Digestive tract tumor | 9 |
| Hepatobiliary tumor | 11 |
| Respiratory system tumor | 9 |
| Gynecolgical tumor | 5 |
| Breast cancer tumor | 4 |
| Urinary system tumor | 3 |
| Total | 50 |

The preparation of the lymphocyte expanded and activated stock solution refers to Embodiment 1.

Cells were counted by using a cell counting plate to determine an expansion ratio. An expanded cell viability was obtained through trypan blue staining and cell counting. A phenotype of expanded cells was detected by flow cytometry, and during detection, an FITC-labeled CD3 antibody and an APC-labeled CD8 antibody were used for determination.

**Table 7**

| Serial number of patients | Gender | Age | Tumor type | Before culture | After culture | | | |
|---|---|---|---|---|---|---|---|---|
| | | | | Number of | Total number | Expansion multiple | Cell viability | Number of CD3+CD8+ |
| | | | | PBMCs (×10⁷) | of cells (×10⁹) | | (%) | cells (×10⁹) |
| 1 | Male | 47 | Lung cancer | 0.91 | 9.12 | 1002.20 | 99.5 | 7.34 |
| 2 | Female | 33 | Leukemia | 0.66 | 6.50 | 984.85 | 98.2 | 5.23 |
| 3 | Female | 59 | Duodenum mesenchymoma | 0.72 | 8.25 | 1145.83 | 98.5 | 6.67 |
| 4 | Female | 67 | Liver cancer | 0.67 | 7.85 | 1171.64 | 99.5 | 6.56 |
| 5 | Male | 47 | Acute lymphoblastic leukemia | 0.80 | 8.14 | 1017.50 | 99.4 | 6.80 |
| 6 | Female | 68 | Gallbladder carcinoma | 0.74 | 7.50 | 1013.51 | 97.5 | 6.35 |
| 7 | Female | 52 | Ovarian cancer | 0.97 | 9.62 | 991.75 | 99.6 | 7.84 |
| 8 | Male | 48 | Leukemia | 0.83 | 9.55 | 1150.60 | 98.2 | 7.83 |
| 9 | Male | 56 | Liver cirrhosis | 0.76 | 8.26 | 1086.84 | 99.5 | 6.83 |
| 10 | Female | 65 | Endometrial cancer | 0.80 | 8.75 | 1093.75 | 99.8 | 7.38 |
| 11 | Male | 76 | Renal cancer | 0.86 | 8.75 | 1017.44 | 98.3 | 7.73 |
| 12 | Male | 83 | Cardiac carcinoma | 0.87 | 9.01 | 1035.63 | 99.6 | 7.23 |
| 13 | Female | 73 | Liver cancer | 0.78 | 8.85 | 1134.62 | 97.7 | 7.37 |
| 14 | Male | 78 | Cancer of biliary duct | 0.75 | 7.47 | 996.00 | 96.8 | 6.32 |
| 15 | Male | 78 | Liver cancer | 0.71 | 7.14 | 1005.63 | 98.3 | 5.73 |
| 16 | Female | 37 | B-acute lymphoblastic leukemia | 0.69 | 8.14 | 1179.71 | 97.7 | 6.72 |
| 17 | Female | 55 | Bladder cancer | 0.90 | 9.79 | 1087.78 | 99.4 | 7.92 |
| 18 | Female | 66 | Breast cancer | 0.86 | 9.84 | 1144.19 | 99.2 | 7.93 |
| 19 | Male | 48 | Non-small cell | 0.63 | 7.56 | 1200.00 | 96.7 | 6.84 |
| | | | lung cancer | | | | | |
| 20 | Male | 56 | Lung cancer | 0.81 | 8.49 | 1048.15 | 99.5 | 6.93 |
| 21 | Female | 47 | Cervical cancer | 0.80 | 8.06 | 1007.50 | 96.9 | 6.68 |
| 22 | Male | 61 | Small cell lung cancer | 0.62 | 6.71 | 1082.26 | 99.1 | 5.64 |
| 23 | Female | 35 | Acute lymphoblastic leukemia | 0.89 | 9.12 | 1024.72 | 99.1 | 7.93 |
| 24 | Female | 58 | Breast cancer | 0.91 | 9.97 | 1095.60 | 99.3 | 8.21 |
| 25 | Male | 65 | Gastric cancer | 0.63 | 6.69 | 1061.90 | 98.9 | 5.25 |
| 26 | Male | 55 | Small cell lung cancer | 0.68 | 7.69 | 1130.88 | 98.9 | 6.29 |
| 27 | Male | 36 | Leukemia | 0.83 | 9.79 | 1179.52 | 98.6 | 7.92 |
| 28 | Female | 55 | Non-small cell lung cancer | 0.57 | 5.88 | 1031.58 | 99.3 | 4.82 |
| 29 | Female | 60 | Colon cancer | 0.72 | 6.62 | 919.44 | 98.8 | 5.83 |
| 30 | Female | 75 | Cervical cancer | 0.83 | 8.89 | 1071.08 | 99.5 | 7.23 |
| 31 | Male | 67 | Rectal cancer | 0.78 | 8.56 | 1097.44 | 99.3 | 6.93 |
| 32 | Male | 56 | Gallbladder carcinoma | 0.72 | 8.69 | 1206.94 | 99.4 | 7.23 |
| 33 | Male | 59 | Esophagus cancer | 0.70 | 7.39 | 1055.71 | 98.8 | 5.93 |
| 34 | Female | 67 | Ovarian cancer | 0.73 | 8.25 | 1130.14 | 99.3 | 6.80 |
| 35 | Female | 71 | Gallbladder carcinoma | 0.53 | 5.16 | 973.58 | 99.6 | 4.42 |
| 36 | Male | 62 | Lung cancer | 0.51 | 4.99 | 978.43 | 98.9 | 4.32 |
| 37 | Female | 42 | Acute lymphoblastic leukemia | 0.79 | 8.69 | 1100.00 | 98.5 | 7.04 |
| 38 | Male | 53 | Gastric cancer | 0.59 | 6.39 | 1083.05 | 98.9 | 5.94 |
| 39 | Female | 68 | Liver cancer | 0.65 | 7.11 | 1093.85 | 98.6 | 5.83 |
| 40 | Male | 56 | Prostate cancer | 0.88 | 9.44 | 1072.73 | 98.7 | 7.83 |
| 41 | Female | 52 | Breast cancer | 0.72 | 8.53 | 1184.72 | 99.7 | 6.93 |
| 42 | Male | 60 | Liver cancer | 0.82 | 10.59 | 1291.46 | 98.9 | 8.73 |
| 43 | Female | 65 | Pancreatic cancer | 0.62 | 7.36 | 1187.10 | 98.8 | 6.02 |
| 44 | Male | 63 | Lung cancer | 0.91 | 9.78 | 1074.73 | 99.6 | 7.93 |
| 45 | Female | 45 | Breast cancer | 0.63 | 6.39 | 1014.29 | 99.6 | 5.70 |
| 46 | Male | 57 | Lung cancer | 0.70 | 7.64 | 1091.43 | 98.9 | 6.53 |
| 47 | Female | 62 | Liver cancer | 1.01 | 10.25 | 1014.85 | 98.7 | 8.32 |
| 48 | Male | 28 | Leukemia | 0.62 | 6.14 | 990.32 | 98.8 | 5.20 |
| 49 | Male | 39 | Acute lymphoblastic leukemia | 0.75 | 8.88 | 1184.00 | 99.7 | 7.30 |
| 50 | Female | 51 | Gastric cancer | 0.67 | 7.40 | 1104.48 | 99.2 | 6.12 |

(2) 787 clinical killing trials were performed using the expanded cells above, and a biological activity KT₅₀ of the cells was detected, which means a time for killing to reach 50%. The shorter the time, the better the biological efficacy. A frequency of KT₅₀ less than 1 h accounts for 2.29%, a frequency of KT₅₀ between 1 h and 2 h accounts for 34.81%, a frequency of KT₅₀ between 2 h and 3 h accounts for 31.77%, a frequency of KT₅₀ between 3 h and 4 h accounts for 18.3%, a frequency of KT₅₀ between 4 h and 5 h accounts for 8.39%, a frequency of KT₅₀ between 5 h and 6 h accounts for 2.92%, and a frequency of KT₅₀ between 6 h and 8 h accounts for 1.52%.

### Experimental Example 1 Study on Activated Expanded Lymphocytes of the Present Disclosure for Immunotherapy

Inclusion criteria for an EAL treatment group are as follows: patients were histologically or cytologically diagnosed as gastric cancer, lung cancer, etc., the expected lifetime was greater than 12 weeks, and a performance status (PS) score by the Eastern Cooperative Oncology Group (ECOG) was in a range from 0 to 2; and patients with an ECOG score greater than 2 points were excluded. All patients agreed to their treatment regimens and signed an informed consent form.

The EAL treatment group: 50 patients, see Embodiment 5 for ages, genders and disease information of the patients, with autologous infusion of expanded activated lymphocyte preparations. Treatment regimen: in a first course of treatment, subjects received 4 intravenous infusions, once per week; in a second course of treatment, the subjects received 4 intravenous infusions, once every two weeks; in a third course of treatment, the subjects received 4 intravenous infusions, once every three weeks; and in a fourth course of treatment, the subjects received 8 intravenous infusions, once every four weeks.

A control group: 20 patients with cancer (five patients for each of gastric cancer, small cell lung cancer, liver cancer and leukemia) who enter hospital treatment at the same stage, and having undergone chemotherapy or radiotherapy and a conventional treatment regimen; and patients with a history of cellular therapy and having the ECOG greater than 2 were excluded. During treatment, a chemotherapy regimen of the study objects include: CE (etoposide+carboplatin), EP (etoposide+cisplatin), IP (irinotecan+cisplatin), PP (paclitaxel+cisplatin/nedaplatin) and DP (docetaxel+cisplatin), CAV (cyclophosphamide+famomycin+vincristine), NP (navelbine+cisplatin), monotherapy with pemetrexed, monotherapy with docetaxel, monotherapy with temozolomide capsules, and monotherapy with etoposide capsules. Patients with incomplete treatment history or missed follow-up were not counted. The effectiveness and safety of cellular immunotherapy was analyzed.

### (1) Efficacy Evaluation

A median OS time in the EAL treatment group was significantly longer than that of the control group. The EAL immunotherapy prolongs the survival of cancer patients for 1-7 years; and improves side effects and adverse reactions such as nausea, vomiting, hair loss, diarrhea, loss of appetite, fever, chills, headache, pruritus, rash, and tachycardias of drug-applied patients caused by chemotherapy, and has no serious adverse reactions such as hemogram reduction and liver and kidney function damage, thereby significantly enhancing and improving the quality of life of the patients.

### (2) Safety Evaluation

The EAL treatment group: EAL-treated patients shown only grade 1 or grade 2 autonomic adverse events (see Table 8); none of the patients shown pulmonary or renal symptoms, infectious symptoms, deterioration of liver function, or autoimmune diseases; and no treatment-related deaths were recorded.

The control group: the patients shown severe adverse reactions such as hemogram reduction and liver and kidney function damage, and had severe adverse reactions such as nausea, vomiting, diarrhea, and hair loss.

**Table 8**

| Adverse event | Patients with immunotherapy |
|---|---|
| Fever | 2 (4.00%) |
| Chill | 1 (2.00%) |
| Headache | 1 (2.00%) |
| Nausea | 0 |
| Pruritus | 0 |
| Rash | 1 (2.00%) |
| Tachycardias | 0 |
| Diarrhea | 0 |

### Experimental Example 2 Study on Drug Combination of Activated Expanded Lymphocytes of the Present Disclosure

### 1. Patient Entry

Clinical treatment studies were conducted in 100 patients with low-risk or mid-risk pediatric acute myelogenous leukemia (AML) with low-level minimal residual disease (MRD).

The low-level MRD was defined as a proportion of leukemic blast cells at each myelopuncture less than 0.5%. High-risk AML, acute promyelocytic leukemia, disease recurrence, low-risk diseases of sustained negative MRD after induction chemotherapy, and patients with MRD greater than 0.5% after 6 months of treatment were excluded. The 100 patients participating in this study were divided into 2 groups according to parents' willingness and economics. A combination treatment group (n=50) received chemotherapy and EAL treatment, and a chemotherapy group (n=50) received chemotherapy alone. See Table 9. The parents or legal guardians of each patient provided written informed consent form for chemotherapy and EAL treatment.

### 2. Treatment Regimen

### (1) Chemotherapy Regimen

Induction chemotherapy includes 7-day 100-150 mg/m² of cytarabine in combination with anthracyclines (2-day 8-10 mg/m² of idarubicin) and 3-day 100-150 mg/m² of etoposide. Consolidation chemotherapy was initiated after 2 cycles of induction. Consolidation was composed of three regimens: regimen 1, high dose cytarabine (Ara-c 3g/m², 3 days) and anthracycline (8-10 mg/m² of idarubicin, 2 days); regimen 2, 7-day 4-6 mg/m² Harringtonine and 7-day 100-150 mg//m² of cytarabine; and regimen 3, 7-day 100-150 mg/m² cytarabine, in combination with anthracyclines (8-10 mg/m² of idarubicin, 2 days) and 3-day 100-150 mg/m² etoposide. The three regimens were used interchangeably for a total of 12-18 months. In each cycle of small doses of Ara-c consolidation chemotherapy, three-way intrathecal chemotherapy (methotrexate, cytarabine, and hydrocortisone) was administered 4-8 times in total to prevent central nervous system leukemia.

### (2) EAL Therapy

EAL treatment was performed on low-risk and mid-risk AML patients whose MRD state was consistently positive (<0.5%), and patients whose MRD state became positive or whose MRD load increased by 1 copy. In this study, patients in the combination treatment group received combination treatment of the EAL treatment and the chemotherapy. The EAL was to collect 10-80 mL peripheral blood (determined from absolute peripheral blood lymphocyte counts) from each patient. Then, PBMC suspension was placed in a flask coated with an immobilized anti-CD3 antibody, and activation, passaging and expansion culture were performed according to a preparation method of Embodiment 1; and lymphocytes were then collected, filtered through a membrane with a pore size of 100 µm and resuspended in 100 mL of normal saline containing 1% human serum albumin for intravenous infusion back to individual patients. A final product was divided into 1-3 infusion bags according to the total number of cells after culturing, and each bag contained 1×10⁹ to 2×10¹⁰ CD3+CD8+T cells.

The EAL was stored in a 15-25°C thermostat, and infused to patients within 12 h, to verify that a cell viability was consistently greater than or equal to 90%. Each blood transfusion contained cells greater than or equal to 3×10⁹. The study regimen was approved by the Ethic Committee. The informed consent of all patient parents or legal guardians has been obtained.

### 3. Monitoring and Tracking

Monitoring Measures: MRD of the core-binding factor AML (CBF AML) was monitored by real-time quantitative reverse transcription polymerase chain reaction (RT-PCR) assays. For non-CBF AML patients, the MRD is detected via flow cytometry according to a leukemia associated immunophenotype (LAIP).

Monitoring and follow-up time points: bone marrow samples were collected at diagnosis, prior to each chemotherapy cycle, then every 3 months to 3 years and every 6 months to 5 years.

### 4. Results

No significant difference between groups were observed among the 100 children participating in the study in terms of gender, age, genetic characteristics, risk stratification, an MRD state after 8 months of chemotherapy, or intensity of chemotherapy.

The combination treatment group included 26 males and 24 females with a median age of 10.5 years old (range, 1-15 years old). Where, there were 42 cases in the low-risk group (84.00%) and 8 cases in the mid-risk group.

At the time of onset, a mean white blood cell count was 27.9×10⁹ (range, 2.1-100×10⁹), two patients suffered from central nervous system leukemia, and one patient appeared with a large mandibular mass. The combination treatment group received four (range, 1-12) EAL treatments on average. In this study, 6-12 months (8 months on average) after the start of chemotherapy was chosen to start the EAL treatment.

50 children of the combination treatment group received a total of 220 EAL blood transfusion (4 blood transfusion per patient on average). Analysis of cell phenotype and cell count for the 220 EAL blood transfusion indicated an average cell count of 7.5±0.5×10⁹AL and an average cell viability of 98.9%. The T cell phenotype was distributed as follows: CD3+, 98.95%; and CD3+CD8+, 87.26%.

The control group included 25 males and 25 females with a median age of 10.6 years old (range, 1-15 years old). Where, 42 people belong to the low-risk group (84.00%), and 8 people belong to the med-risk group. At the time of onset, one patient suffered from central nervous system leukemia, and one patient suffered from extensive thoracolumbar infiltration.

**Table 9**

| | Combination treatment group | Chemotherapy group |
|---|---|---|
| N | 50 | 50 |
| Age (years old) | | |
| ≥ 10 years old (n) | 28 | 30 |
| < 10 years old (n) | 26 | 20 |
| Gender | | |
| Male (n) | 26 | 25 |
| Female (n) | 24 | 25 |
| Risk stratification | | |
| Low risk (n) | 42 | 42 |
| Mid risk (n) | 8 | 8 |
| Chemotherapy cycle (n) | 9.10±0.38 | 10.18±0.58 |
| MRD after 8 months of CBF-AML chemotherapy (%) | 0.05±0.02 | 0.05±0.11 |
| MRD after 8 months of non-CBFAML chemotherapy (%) | 0.08±0.20 | 0.06±0.31 |

### (2) Negative rates of MRD in two groups during consolidation therapy

EAL treatment began when the MRD before treatment was less than 0.5% and the average MRD load was 0.055%. After EAL treatment and chemotherapy, 49 out of 50 patients (98.00%) had an MRD state turned negative, but one patient's MRD state remained positive after combination treatment. In the combination treatment group, an average time for the MRD state to stay negative during consolidation chemotherapy was 7.9 months.

In the control group, the MRD state of 30 patients (60.00%) turned negative. An average time for the MRD state to stay negative during consolidation chemotherapy was 4.8 months.

98.00% of patients in the combination treatment group had the MRD state turned negative during consolidation chemotherapy, which was significantly higher than that of the patients in the control group. The MRD state of the combination treatment group remained negative for 7.9 months, which was significantly longer than that of the chemotherapy group.

### (3) Comparison of survival rates between two groups

A median EFS in the combination treatment group was 81 months, while a median EFS in the control group was 50 months.

An average 5-year EFS rate in the combination treatment group was 88.9%, while an average 5-year EFS rate in the control group was 69.7%.

In the combination treatment group, four patients (8.00%) experienced disease recurrence. Among them, 3 patients experienced bone marrow recurrence, and 1 patient experienced bone marrow recurrence after central nervous system recurrence.

In the control group, 12 patients (24.0%) experienced disease recurrence, all of which were bone marrow relapses. A median time of recurrence is 15.3 months (range, 6-34). A median recurrence time in the combination treatment group was 35.8 months, which is significantly longer than that in the chemotherapy group.

### (4) Adverse reactions

The combination treatment group occasionally experienced fever or chills, and in the treatment process, no severe organ damage caused by inflammatory cytokine cascade was observed, nor was there any cell treatment-related death.

The above description of the specific implementations of the present disclosure does not limit the present disclosure, and those skilled in the art can make various changes or modifications according to the present disclosure, and all these changes and modifications shall belong to the scope of protection of the claims of the present disclosure as long as they do not depart from the spirit of the present disclosure.

## Claims

1. A method for preparing an activated expanded lymphocyte stock solution, comprising the following steps:
(1) extracting peripheral blood mononuclear cells (PBMCs) via centrifugation of autologous peripheral whole blood, and co-culturing the peripheral blood mononuclear cells with a lymphocyte activator in a serum-free medium to perform lymphocyte culture activation, producing cultured activated lymphocytes, wherein the peripheral blood mononuclear cells are cultured and activated in the serum-free medium at a starting density of (0.2-1.6)×10⁶ cells/mL and an activation culture temperature of 37.0°C±1.0°C in an activation culture environment that contains 7.5%±1.0% CO₂, wherein the lymphocyte activator is selected from the group consisting of an anti-human CD2 antibody, an anti-human CD3 antibody, an anti-human CD28 antibody, phytohemagglutinin (PHA) and any combination thereof, or is a carrier coated the antibody, and wherein the serum-free medium is selected from the group consisting of KBM 581, GT-T551 H3 and any combination thereof;
(2) placing the cultured activated lymphocytes prepared in step (1) into a serum-free medium selected from the group consisting of KBM 581, GT-T551 H3 and any combination thereof at a cell density of (0.5-5)×10⁶ cells/mL to perform passage culture for 1 to 5 generations at a temperature of 37.0°C±1.0°C in an activation culture environment that contains 7.5%±1.0% CO₂, producing a lymphocyte activation culture;
(3) adding a serum-free medium selected from the group consisting of KBM 581, GT-T551 H3 and any combination thereof at an amount 5 to 10 times the volume of the lymphocyte activation culture to the lymphocyte activation culture obtained in step (2) to perform expansion culture for 1 to 5 generations at a temperature of 37.0°C±1.0°C in an activation culture environment that contains 7.5%±1.0% CO₂; and
(4) centrifuging, washing, and collecting the activated expanded lymphocytes.

2. The method according to claim 1, wherein said extracting peripheral blood mononuclear cells (PBMCs) via centrifugation of autologous peripheral whole blood comprises the following steps:
adding a separation medium and a diluent to whole blood subjected to anti-coagulation treatment, stirring, mixing uniformly to obtain a mixture, adding the mixture to a separation solution, centrifuging at a speed of 1000-3000 rpm for a period of 10-40 min, collecting a cell layer between interfaces, adding a washing solution, centrifuging, washing and collecting cells to obtain the peripheral blood mononuclear cells, wherein the separation medium is selected from the group consisting of a hydroxyethyl starch 40 sodium chloride injection (HES), Percoll, Ficoll-Paque PLUS and any combination thereof, a volume ratio of the whole blood : the diluent is 1:(1-2), the diluent is selected from the group consisting of a sodium chloride injection, a Hank's buffer, a Lactated Ringer's solution, a Dulbecco's phosphate buffer and any combination thereof, the separation solution is selected from the group consisting of a hydroxyethyl starch 40 sodium chloride injection (HES), Ficoll, Lymphoprep, Lymphocyte Separation Media, Cell Separation Media and any combination thereof, an osmotic pressure of the separation solution ranges from 300 mOsmol/kg to 360 mOsmol/kg, and the washing solution is selected from the group consisting of a 0.1% human albumin sodium chloride injection, a Dulbecco's phosphate buffer, a sodium chloride injection and any combination thereof.

3. The method according to any one of claims 1 to 2, wherein the centrifugation is performed at a speed of 1500-2500 rpm for a period of 15-30 min, preferably at a speed of 2000-2500 rpm for a period of 20-25 min, and wherein the washing is centrifugal washing performed for 1 to 5 times at a speed of 500-2000 rpm for a period of 5-20 min, preferably performed for 2 to 3 times at a speed of 1000-1800 rpm for a period of 10-15 min.

4. The method according to any one of claims 1 to 3, wherein 300-600 IU/mL, preferably 400-500 IU/mL of cytokines IL-2 are optionally added to the serum-free medium.

5. The method according to any one of claims 1 to 4, wherein an expansion multiple of the activated expanded lymphocytes is greater than or equal to 900 times, preferably greater than or equal to 1,000 times, and more preferably, greater than or equal to 1,100 times.

6. The method according to any one of claims 1 to 5, wherein the activated expanded lymphocytes have a viability that is greater than or equal to 95%, preferably greater than or equal to 98%.

7. The method according to any one of claims 1 to 6, wherein the activated expanded lymphocytes comprise CD8+T cells at an amount of greater than or equal to 1×10⁹, preferably from 1×10⁹ to 2×10¹⁰, and more preferably from 4×10⁹ to 9.5×10⁹.

8. The method according to any one of claims 1 to 7, wherein the activated expanded lymphocytes have a biological activity KT₅₀ of less than or equal to 8.5, preferably less than or equal to 4, and more preferably less than or equal to 0.7141.

9. A pharmaceutical composition comprising an activated expanded lymphocyte stock solution prepared by the method according to any one of claims 1 to 8, wherein thepharmaceutical composition consists of (1-20)×10⁷/mL activated expanded lymphocytes, 0.5-2% human albumin and saline for injection, and has an activated expanded lymphocyte viability of greater than or equal to 95%, wherein CD8+T cells in the activated expanded lymphocytes are (1-20)×10⁷/mL, and wherein the pharmaceutical composition is free of preservatives and antibiotics.

10. The pharmaceutical composition according to claim 9, wherein a cell expansion multiple of a biological sample is greater than or equal to 900 times, preferably greater than or equal to 1,000 times, and more preferably greater than or equal to 1,100 times.

11. The pharmaceutical composition according to any one of claims 9 to 10, wherein the activated expanded lymphocytes have a biological activity KT₅₀ of less than or equal to 8.5, preferably less than or equal to 4, and more preferably less than or equal to 0.7141.

12. The pharmaceutical composition according to any one of claims 9 to 11, wherein the total number of the activated expanded lymphocytes in single reinfusion of a patient is less than or equal to 2×10¹⁰, and a cell viability within a validity period is greater than or equal to 85%.

13. The pharmaceutical composition according to any one of claims 9 to 12, wherein an activated expanded lymphocyte viability of the composition stored at 15-25°C within 12 h is greater than or equal to 85%.

14. A method for preparing the pharmaceutical composition comprising the activated expanded lymphocyte stock solution according to any one of claims 9 to 13, comprising resuspending the activated expanded lymphocyte stock solution in saline for injection containing human albumin.

15. A method for immunotherapy using the activated expanded lymphocytes prepared by the method according to any one of claims 1 to 8, comprising the following steps:
(1) obtaining a biological sample containing lymphocytes from an individual;
(2) activating, passaging and expanding the lymphocytes by using the preparation method of the present disclosure to obtain the activated expanded lymphocytes; and
(3) reinfusing the activated expanded lymphocytes or a pharmaceutical composition thereof to the individual.

16. A dosage regimen for immunotherapy using the activated expanded lymphocytes prepared by the method according to any one of claims 1 to 8, comprising the following regimens:
(1) in a first course of treatment, allowing a subject receiving 3-6 intravenous infusions, once per week;
(2) in a second course of treatment, allowing the subject receiving 3-6 intravenous infusions, once every two weeks;
(3) in a third course of treatment, allowing the subject receiving 3-6 intravenous infusions, once every three weeks; and
(4) in a fourth course of treatment, allowing the subject receiving 7-10 intravenous infusions, once every four weeks.

17. The dosage regimen according to claim 16, comprising the following regimens:
(1) in the first course of treatment, allowing the subject receiving 4-5 intravenous infusions, once per week;
(2) in the second course of treatment, allowing the subject receiving 4-5 intravenous infusions, once every two weeks;
(3) in the third course of treatment, allowing the subject receiving 4-5 intravenous infusions, once every three weeks; and
(4) in the fourth course of treatment, allowing the subject receiving 8-9 intravenous infusions, once every four weeks.

18. The dosage regimen according to any one of claims 15 to 17, wherein the number of the activated expanded lymphocytes per intravenous infusion is greater than or equal to 2×10⁸, preferably greater than or equal to (2-10)×10⁹.

19. Use of the activated expanded lymphocytes prepared by the method according to any one of claims 1 to 8 in the preparation of any one of drugs for anti-tumor immunotherapy, drugs for enhancing antiviral capability and drugs for enhancing treatment of autoimmune diseases.

20. Use of the activated expanded lymphocytes, prepared by the method according to any one of claims 1 to 8, combined with any one of surgery, radiotherapy and chemotherapy in anti-tumor or immunotherapy thereof.
